# EUROPEAN PATENT APPLICATION

(11) **EP 1 878 789 A1**
(43) Date of publication of application: **16.01.2008**
(21) Application number: 06731862.6
(22) Date of filing: 14.04.2006
(51) Int. Cl.: C12N 5/06, A61K 35/14, A61K 35/48, A61L 27/00, A61P 9/00, A61P 9/10, A61P 9/12, C12N 5/02

(54) **FAT-DERIVED PROGENITOR CELL AND USE THEREOF**

(30) Priority: 14.04.2005 JP 2005117588
(71) Applicant: Japan as Represented by General Director of Agency, Suita-shi, Osaka 5658565 (JP); Hubit Genomix, Inc., Tokyo 102-0092 (JP)
(72) Inventor: NAGAYA, Noritoshi, National Cardiovascular Center, Suita-shi, Osaka 565-8565 (JP); MIYATAKE, Kunio, Nat. Org. Osaka Minami Med. Ctr., Kawachinagano-shi, Osaka 586-8521 (JP); KANGAWA, Kenji, National Cardiovascular Center, Suita-shi, Osaka 565-8565 (JP); KATAOKA, Masaharu, National Cardiovascular Center, Suita-shi, Osaka 565-8565 (JP)
(74) Representative: Bosch, Matthias
(86) International application number: PCT/JP2006/307926
(87) International publication number: WO 2006/112390

(57) **Abstract**

[Problems to be solved] An objective of the present invention is to provide methods for producing progenitor cells which show a high proliferation ability *ex vivo* and promote neovascularization or myocardium regeneration, by culturing pluripotent cells in a medium containing adrenomedullin (AM) and/or vascular endothelial growth factor (VEGF). Moreover, another objective of the present invention is to provide the progenitor cells and uses thereof.

[Means for Solving the Problems] In order to solve the above problems, the inventors of the present application have successfully developed a novel culture technique for producing progenitor cells from adipose tissues more efficiently compared to the conventional techniques, by using adrenomedullin (AM) and/or vascular endothelial growth factor (VEGF). The progenitor cells produced by the present inventors are specialized cells having a potent proliferation ability, a revascularization ability, and a myocardium regeneration ability, and have the property of not differentiating into adipocytes even if subjected to differentiation induction into adipocytes. Therefore, the progenitor cells of the present invention can be effective pharmaceutical agents for intractable diseases in the brain, lung, liver, kidney and the like.

## Description

### Technical Field

The present invention relates to progenitor cells derived from adipose tissues, methods for producing the cells, and the use of these cells.

### Background Art

Although rare, pulmonary artery hypertension is a fatal disease characterized by progressive pulmonary hypertension and right heart failure (Non Patent Document 1). Pulmonary vascular endothelial dysfunction is considered to play an important role in the onset of such pulmonary hypertensions. Therefore, the pulmonary vascular endothelium is considered to be a therapeutic target for treating pulmonary hypertensions including primary pulmonary hypertension (Non Patent Document 2).

Ischemic heart diseases (such as angina pectoris and myocardial infarction) are states where the myocardium falls into ischemia or necrosis due to the narrowing or blockage of coronary arteries. Old myocardial infarction is an important causative factor of chronic heart failure. Pharmacotherapies aiming at preventing arrhythmia, recurrence of myocardial infarction, progress of arteriosclerosis, and the like, are applied against chronic heart failure, but there is no effective therapeutic method for restoring necrotised myocardium. Recently, attempts for improving cardiac functions have started where a granulocyte colony stimulating factor is used for mobilizing myeloid cells to the myocardial damage site to promote the regeneration of blood vessels and myocardium. However, it has not been established as a therapeutic agent due to the problems of side effects and the like.

Vascular endothelial progenitor cells are known to exist in the bone marrow/adult peripheral blood/cord blood (Non Patent Document 3), and revascularization treatment using vascular endothelial progenitor cells are currently carried out. These vascular endothelial progenitor cells are known to move from the bone marrow to peripheral blood in response to tissue ischemia or traumatic injury, then migrate to the injured vascular endothelial site, and differentiate into mature endothelial cells therein (Non Patent Documents 4 to 6). Furthermore, the present inventors and other researchers have reported that administration of vascular endothelial progenitor cells improves monocrotaline (hereafter, may be expressed as MCT)-induced pulmonary hypertension (Non Patent Documents 7 and 8).

However, since vascular endothelial progenitor cells transplanted in conventional stem cell transplantation methods are poor in proliferation ability, it has been considered difficult to maintain the required number of these cells by means of *ex vivo* culture. Moreover, it has been reported that patients with inflammatory diseases such as ischemic heart disease/peripheral artery obstruction/pulmonary hypertension, and the like have decreased *in vivo* vascular endothelial progenitor cell functions and cell viability/differentiation ability.

In order to collect cell numbers required for therapy, 600 to 800 ml of bone marrow fluid or a large amount of peripheral blood has to be collected from a patient, making it a highly invasive, risky procedure for the patient. Therefore, obtaining a sufficient number of vascular endothelial progenitor cells was considered difficult. Accordingly, the development of graft cells that can substitute for conventional progenitor cells has been required.

Recently, adipose tissues are attracting attention as a resource of multipotent stem cells (Non Patent Document 9). It is known that the adipose tissue consists of mature adipocytes and adipose stromal cells, and the latter can be differentiated into a variety of cell lineages (Non Patent Documents 10 to 17). The adipose stromal cells are adhesive and can proliferate in culture. Therefore, a large amount of adipose stromal cells can be readily obtained from a small piece of adipose tissue by means of culture (Non Patent Document 18). However, no culture technique for efficiently inducing vascular endothelial progenitor cells from adipocytes has been established.

Information on prior art documents related to the invention of the present application is shown below.
[Non-patent Document 1] Rich S, et al. Ann Intern Med. 1987 ; 107 : 216-223.
[Non-patent Document 2] Archer S, et al. Circulation. 2000 ; 102 : 2781-2791.
[Non-patent Document 3] Asahara T, et al. Science. 1997 ; 275 : 965-967.
[Non-patent Document 4] Takahashi T, et al. Nat Med. 1999; 5 : 434-438.
[Non-patent Document 5] Shintani S, et al. Circulation. 2001 ; 103 : 897-903.
[Non-patent Document 6] Shintani S, et al. Circulation. 2001 ; 103 : 2776-2779.
[Non-patent Document 7] Nagaya N, et al. Circulation. 2003 ; 108 : 889-895.
[Non-patent Document 8] Takahashi M, et al. Tissue Eng. 2004 ; 10 : 771-779.
[Non-patent Document 9] Zuk PA, et al. Mol Biol Cell. 2002 ; 13 : 4279-4295.
[Non-patent Document 10] Zuk PA, et al. Tissue Eng. 2001 ; 7 : 211-228.
[Non-patent Document 11] Hicok KC, et al. Tissue Eng. 2004 ; 10 : 371-380.
[Non-patent Document 12] Erickson GR, et al. Biochem Biophys Res Commun. 2002 ; 290 : 763-769.
[Non-patent Document 13] Cousin B, et al. Biochem Biophys Res Commun. 2003 ; 301 : 1016-1022.
[Non-patent Document 14] Safford KM, et al. Biochem Biophys Res Commun. 2002 ; 294 : 371-379.
[Non-patent Document 15] Miranville A, et al. Circulation. 2004 ; 110 : 349-355.
[Non-patent Document 16] Planat-Benard V, et al. Circ Res. 2004 ; 94 : 223-229.
[Non-patent Document 17] Planat-Benard V, et al. Circulation. 2004 ; 109 : 656-663.
[Non-patent Document 18] Ailhaud G,, et al. Annu Rev Nutr. 1992 ; 12 : 207-233.
[Non-patent Document 19] Kitamura K, et al. Biochem Biophys Res Commun. 1993 ; 192 : 553-560.
[Non-patent Document 20] Nagaya N, et al. Peptides. 2004 ; 25 : 2013-2018.
[Non-patent Document 21] Nagaya N, et al. Circulation. 2000 ; 101 : 498-503.
[Non-patent Document 22] Nagaya N, et al. Circulation. 2004 ; 109 : 351-356.
[Non-patent Document 23] Nakamura R, et al. Circulation. 2004 ; 110 : 426-431.
[Non-patent Document 24] Tokunaga N, et al. Circulation. 2004 ; 109 : 526-531.
[Non-patent Document 25] Okumura H, et al. Circulation. 2004 ; 109 : 242-248.
[Non-patent Document 26] Okumura H, et al. Hypertens Res. 2003 ; 26 : S99-S104.
[Non-patent Document 27] Zachary I. Am J Physiol Cell Physiol. 2001 ; 280 : C1375-C1386.
[Non-patent Document 28] Gehling UM, et al. Blood. 2000 ; 95 : 3106-3112.
[Non-patent Document 29] Dimmeler S, et al. J Clin Invest. 2001 ; 108 : 391-397.
[Non-patent Document 30] Oswald J, et al. Stem Cell. 2004 ; 22 : 377-384.

### Disclosure of the Invention

### [Problems to be Solved by the Invention]

Adrenomedullin (hereafter, may be expressed as AM) is a potent vasodilative peptide, which was originally isolated from human pheochromocytoma (Non Patent Documents 19 to 23). AM has been shown to induce angiogenesis at least partially via the phosphatidylinositol 3-kinase (PI3K)/Akt pathway (Non Patent Documents 24 to 26). Moreover, it is known that the neovascularization-promoting ability of AM is not associated with that of the vascular endothelial growth factor (hereafter, may be expressed as VEGF) (Non Patent Documents 27 to 30). Until now, it has not been examined how the concomitant use of AM and VEGF effects the differentiation of adipose stromal cells into endothelial cell lineages or myocardial cell lineages.

The present invention was achieved in view of the above circumstances. An objective of the present invention is to provide methods for producing progenitor cells that show a high proliferation ability *ex vivo* and promote neovascularization or myocardium regeneration, by culturing pluripotent cells in a medium containing adrenomedullin (AM) and/or vascular endothelial growth factor (VEGF). Moreover, another objective of the present invention is to provide cells, or pharmaceutical agents comprising the cells as an active ingredient, which regenerate or repair damaged tissues. Yet another objective of the present invention is to provide methods for regenerating or repairing damaged tissues in subjects, comprising the step of administering the cells to the subjects.

### [Means for Solving the Problems]

In order to solve the above problems, first, the present inventors cultured adipose stromal cells collected from abdominal subcutaneous adipose tissues of rats, together with adrenomedullin (AM) and/or vascular endothelial growth factor (VEGF). As a result, it was revealed that the cultured cells expressed endothelial cell lineage markers such as vascular endothelial cadherin and CD31, and furthermore, the concomitant use of AM and VEGF enhanced the vascular endothelial differentiation of adipose stromal cells via the PI3K signaling pathway.

Next, an investigation on the cellular function of adipose stromal cell-derived progenitor cells revealed that in a matrigel assay, the single use of either AM or VEGF enhances the neovascularization-promoting ability of adipose stromal cells, and the concomitant use of AM and VEGF further enhances the neovascularization-promoting ability of adipose stromal cells as compared to the single use of either AM or VEGF.

Moreover, it was found by an adipocyte formation assay that adipose stromal cells cultured without adding AM or VEGF easily differentiate into adipocytes when adipocyte formation is induced, whereas adipose stromal cells cultured together with AM and/or VEGF hardly differentiate into adipocytes even if adipocyte formation is induced.

Furthermore, the present inventors also examined the effect of progenitor cells of the present invention on pulmonary hypertension model rats generated using monocrotaline (MCT). Three days after MCT injection, adipose-derived progenitor cells were intravenously administered, which revealed that some progenitor cells are incorporated into pulmonary vessels and participate in pulmonary vascularization. It was also revealed that the administration of cells of the present invention into MCT rats attenuates the decrease in the pulmonary capillary density, significantly restores MCT-induced pulmonary hypertension, and improves the survival time of the MCT rats.

Moreover, it was also revealed that the administration of progenitor cells of the present invention into myocardial infarction model rats shows a reduction in the myocardial infarct area and improves hemodynamics. It was confirmed that progenitor cells of the present invention start self-beating when cocultured with myocardial cells from a neonatal rat. Furthermore, the use of a myocardium-specific marker clearly showed the differentiation of progenitor cells of the present invention into myocardial cells.

Namely, the present inventors successfully developed a novel culture technique for producing progenitor cells that can be differentiated into both vascular endothelial cell lineage and myocardial cell lineage, from adipose tissue and more efficiently compared to the conventional techniques, by using the circulation regulatory peptide adrenomedullin (AM), and thereby completed the present invention. The progenitor cells produced by the present inventors are specialized cells having a potent proliferation ability, a revascularization ability, and a myocardium regeneration ability. Furthermore, these cells have the property of not becoming adipocytes even if they are subjected to adipocyte differentiation induction, and therefore can be an effective pharmaceutical agents for intractable diseases in the brain, lung, liver, kidney or the like.

That is, the present invention provides the following [1] to [15]:
[1] a method for producing a progenitor cell, comprising the step of culturing a pluripotent cell in a medium containing a growth factor of any one of the following (i) to (iii):
   (i) adrenomedullin (AM);
   (ii) vascular endothelial growth factor (VEGF); and
   (iii) adrenomedullin (AM) and vascular endothelial growth factor (VEGF);
[2] the method of [1], wherein the pluripotent cell is an adipose stromal cell, an embryonic stem cell, an adult stem cell, a mesenchymal stem cell, a cord blood cell, or a cell which has artificially acquired a pluripotency;
[3] the method of [1], wherein the pluripotent cell is an adipose stromal cell contained in an adipose tissue;
[4] the method of any one of [1] to [3], wherein the pluripotent cell is derived from a mammal;
[5] a progenitor cell produced by a method of any one of [1] to [4];
[6] a method for regenerating or repairing a damaged tissue in a subject, comprising the step of administering the progenitor cell of [5] to the subject;
[7] a method for newly generating or regenerating a peripheral vessel of a damaged tissue in a subject, comprising the step of administering the progenitor cell of [5] to the subject;
[8] a method for newly generating or regenerating a cell of a damaged tissue in a subject, comprising the step of administering the progenitor cell of [5] to the subject;
[9] the method of any one of [6] to [8], comprising the step of administering a growth factor simultaneously with a progenitor cell to the subject;
[10] the method of [9], wherein the growth factor is any one of following (i) to (iii):
   (i) adrenomedullin (AM);
   (ii) vascular endothelial growth factor (VEGF); and
   (iii) adrenomedullin (AM) and vascular endothelial growth factor (VEGF);
[11] the method of any one of [6] to [10], wherein the damaged tissue is a tissue damaged by an ischemic heart disease or pulmonary hypertension;
[12] a pharmaceutical agent which regenerates or repairs a damaged tissue, comprising the progenitor cell of [5] as an active ingredient;
[13] a pharmaceutical agent which newly generates or regenerates a peripheral vessel of a damaged tissue, comprising the progenitor cell of [5] as an active ingredient;
[14] a pharmaceutical agent which newly generates or regenerates a cell of a damaged tissue, comprising the progenitor cell of [5] as an active ingredient; and
[15] the pharmaceutical agent of any one of [12] to [14] wherein the damaged tissue is a tissue damaged by an ischemic heart disease, peripheral artery obstruction, or pulmonary hypertension.

### Brief Description of the Drawings

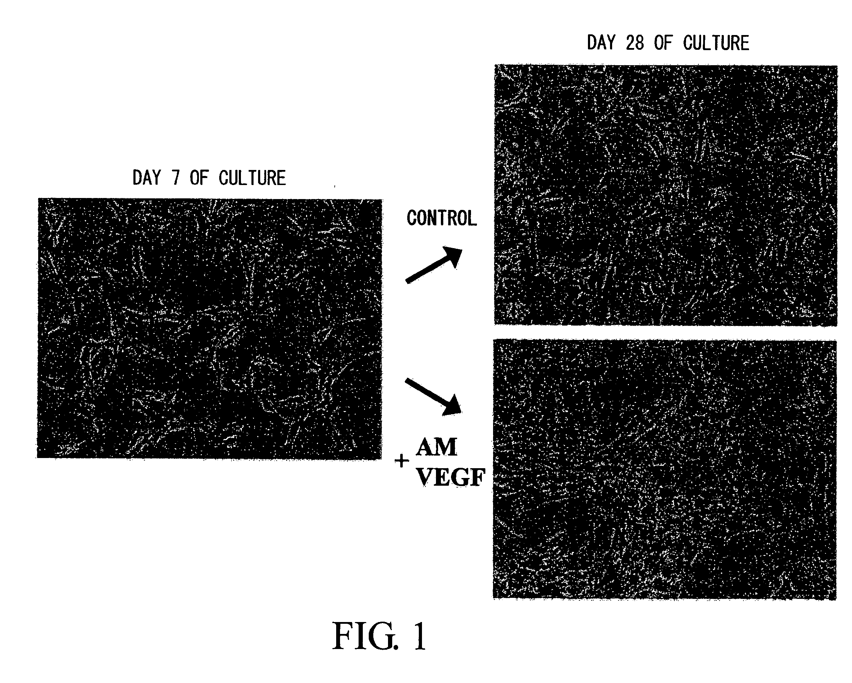

Fig. 1 shows photographs depicting the morphological change of adipose stromal cells. Adipose stromal cells on day 7 of culture showed a spindle-shape, and this shape was maintained without change up to day 28 in the control medium. Adipose stromal cells cultured together with adrenomedullin (AM) and vascular endothelial growth factor (VEGF) exhibited a cobblestone appearance by day 28. Scale bar: 100 µm.

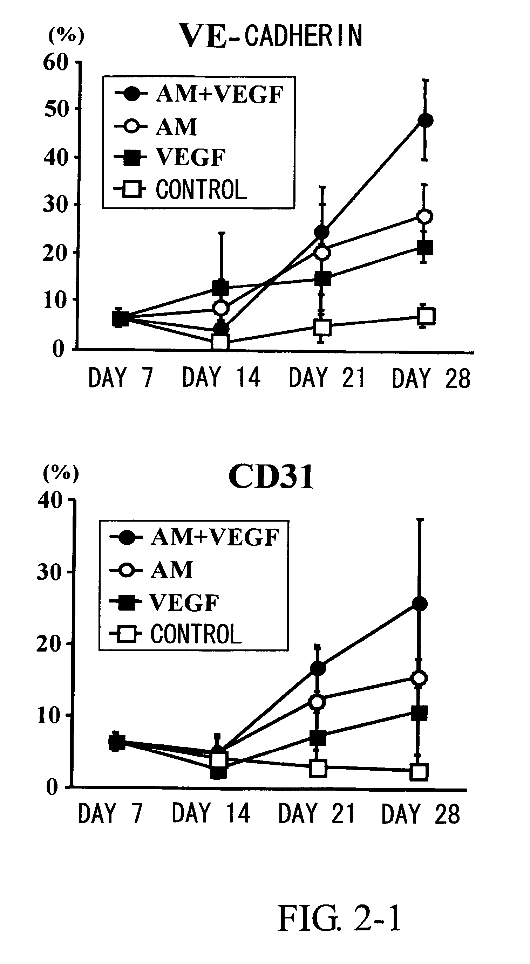

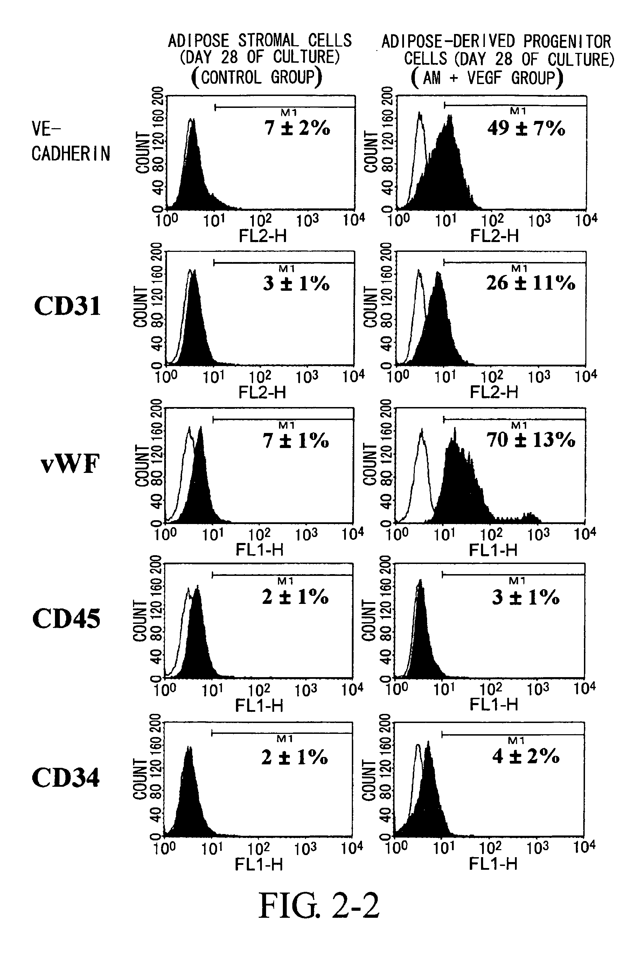

Fig. 2-1 shows the analytical results of the characteristics of adipose stromal cells in the 28-day culture period, using a fluorescence-activated cell sorter (FACS). Adipose stromal cells expressing endothelial cell-specific markers such as vascular endothelial (VE)-cadherin (A) and CD31 (B) gradually increased in the AM group, the VEGF group, and the AM + VEGF group, whereas such cells were not increased in the control group.
Fig. 2-2 shows the expression ofVE-cadherin, CD31, von Willebrand factor (vWF), CD34, and CD45 in the control group and the AM + VEGF group on day 28. Values are means ±SEM.

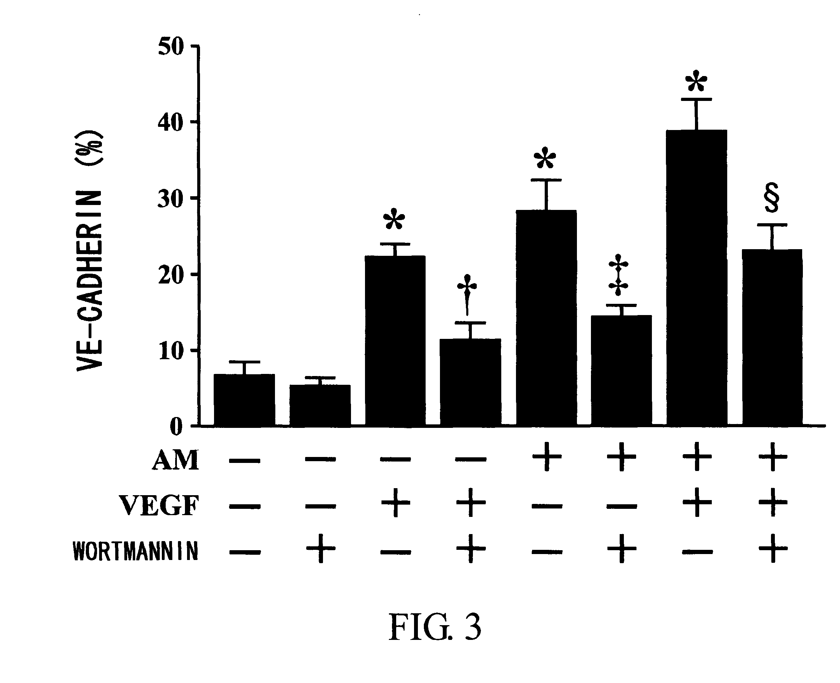

Fig. 3 shows that AM and VEGF induces the endothelial differentiation of adipose stromal cells at least partially via the phosphatidylinositol 3-kinase signaling pathway. Adipose stromal cells were cultured in the presence or absence of wortmannin from day 21 to day 28, and the expression of VE-cadherin thereof was analyzed by FACS on day 28. Values are means ± SEM. * P<0.05, as compared to control; † P<0.05, as compared to VEGF; ‡P<0.05, as compared to AM; § P<0.05, as compared to AM + VEGF.

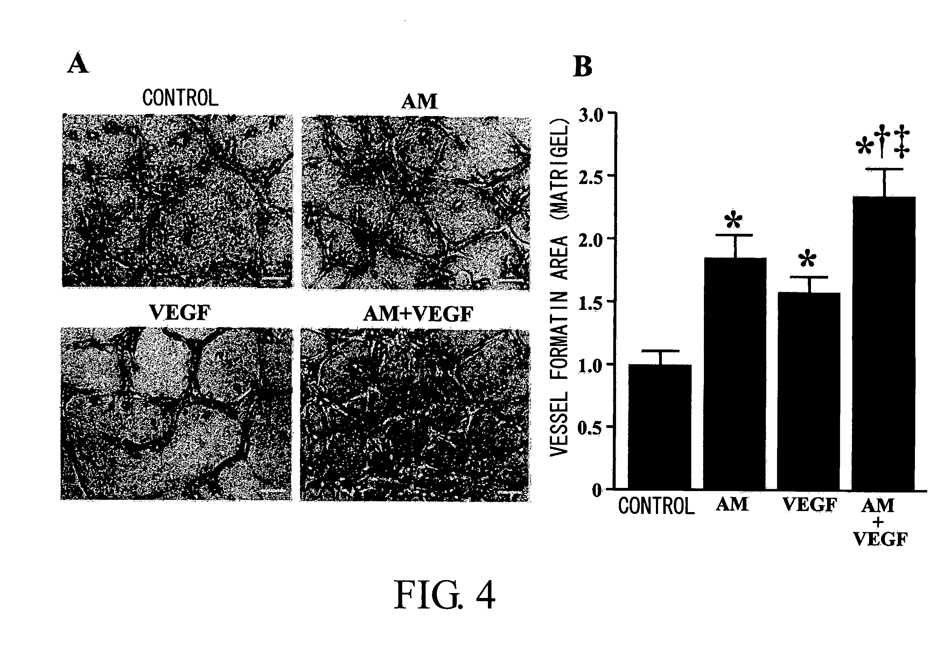

Fig. 4 shows photographs and a graph depicting the measurement of neovascularization in a matrigel. A shows representative photographs of vessel formation. Scale bar: 100 µm. B shows the quantitative analysis of the vessel-formed area. In this case, the value of the control group was set to 1.0. Values are means ± SEM. * P<0.05, as compared to control; † P<0.05, as compared to AM; ‡ P<0.05, as compared to VEGF.

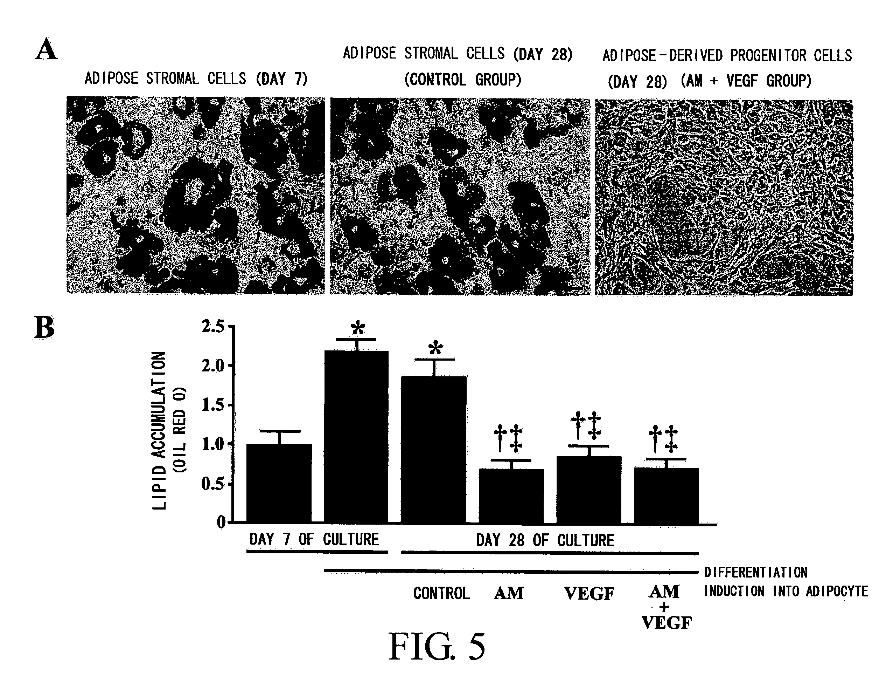

Fig. 5 shows photographs and a graph depicting the measurement of adipocyte formation. A shows a representative photograph of adipose conversion of adipose stromal cells, which were stained with an oil red O solution on day 7, and photographs of the control group and the AM + VEGF group on day 28. Scale bar: 100 µm. B is a graph showing the quantitative analysis of the adipose conversion. The value for adipose stromal cells which did not form adipocytes on day 7 was set to 1.0. Values are means ± SEM. * P<0.05, as compared to adipose stromal cells which did not form adipocytes on day 7; †P<0.05, as compared to adipose stromal cells which formed adipocytes on day 7; ‡ P<0.05, compared adipose stromal cells which formed adipocytes on day 28 with control.

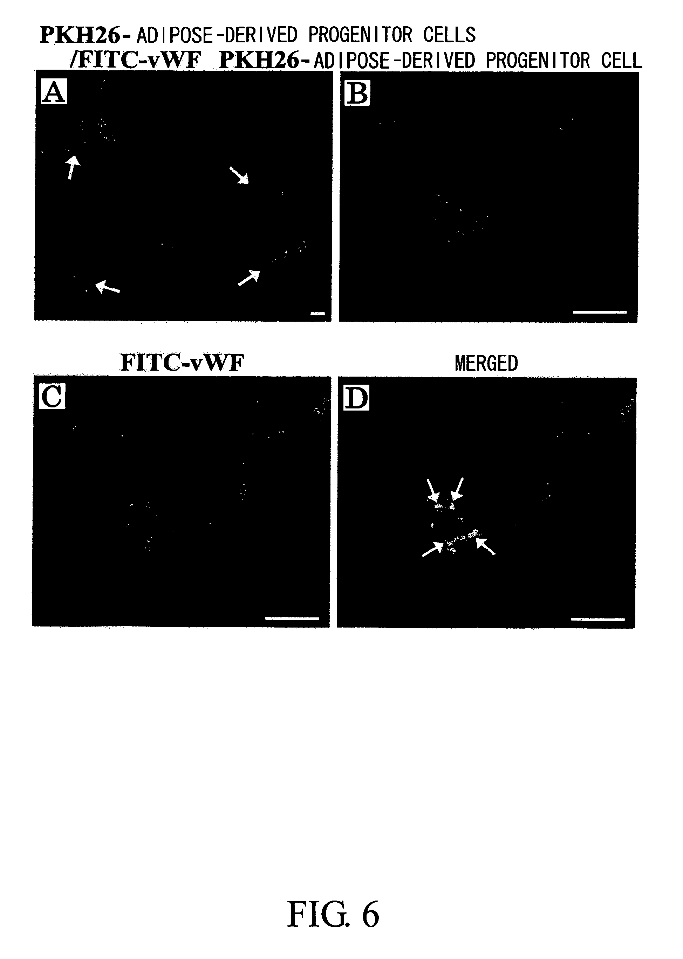

Fig. 6 shows photographs depicting the incorporation, distribution, and differentiation of adipose-derived progenitor cells in the lung of a monocrotaline (MCT)-administered rat (MCT rat) *in vivo.* A shows that fluorescence (PKH26)-labeled adipose-derived progenitor cells that had been intravenously administered were incorporated into the pulmonary arteriolar wall. The arrows show the fluorescence-labeled adipose-derived progenitor cells. B shows cell nuclei by DAPI staining. C shows that the transplanted PKH26-labeled adipose-derived progenitor cells were distributed in pulmonary tissue. The pulmonary vessels were detected by FITC-conjugated anti-vWF. D is a representative photograph of endothelial regeneration. PKH26/vWF double-positive cells are observed between pulmonary alveoli, which were also stained with DAPI. The arrows show PKH26/vWF double-positive cells. Scale bar: 20 µm.

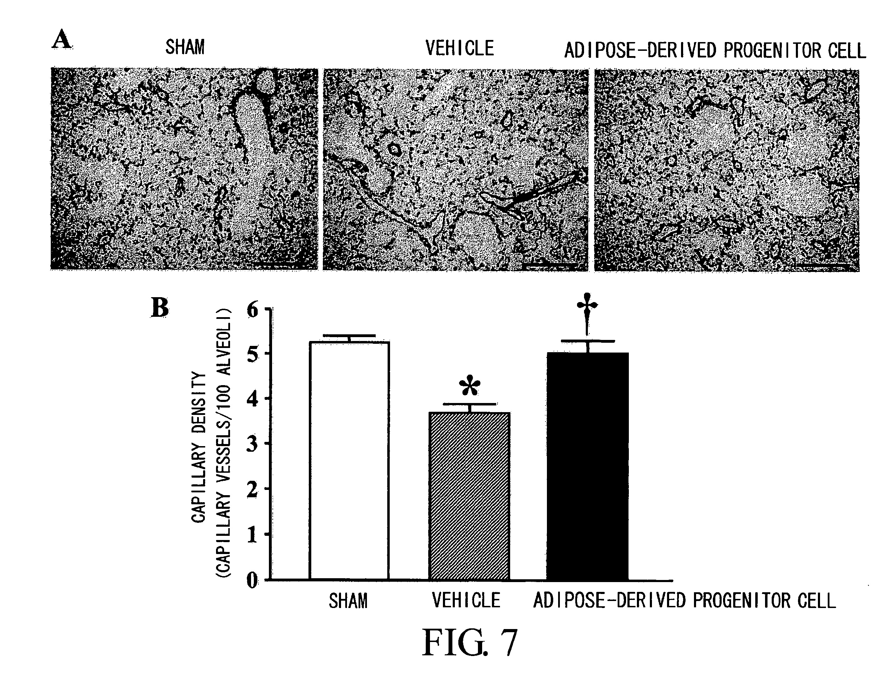

Fig. 7A shows the presence of vWF as detected by the immunohistochemical method in the lung of a sham-treated rat administered with a medium (sham-treated group), a MCT rat administered with a medium (vehicle group), and a MCT rat administered with adipose-derived progenitor cells (adipose-derived progenitor cell group). The capillary density in the adipose-derived progenitor cell group was significantly higher than that of the vehicle group. Scale bar: 100 µm. B is a graph showing the quantitative analysis of the capillary density. Values are means ± SEM. * P<0.05, as compared to sham; † P<0.05, as compared to vehicle.

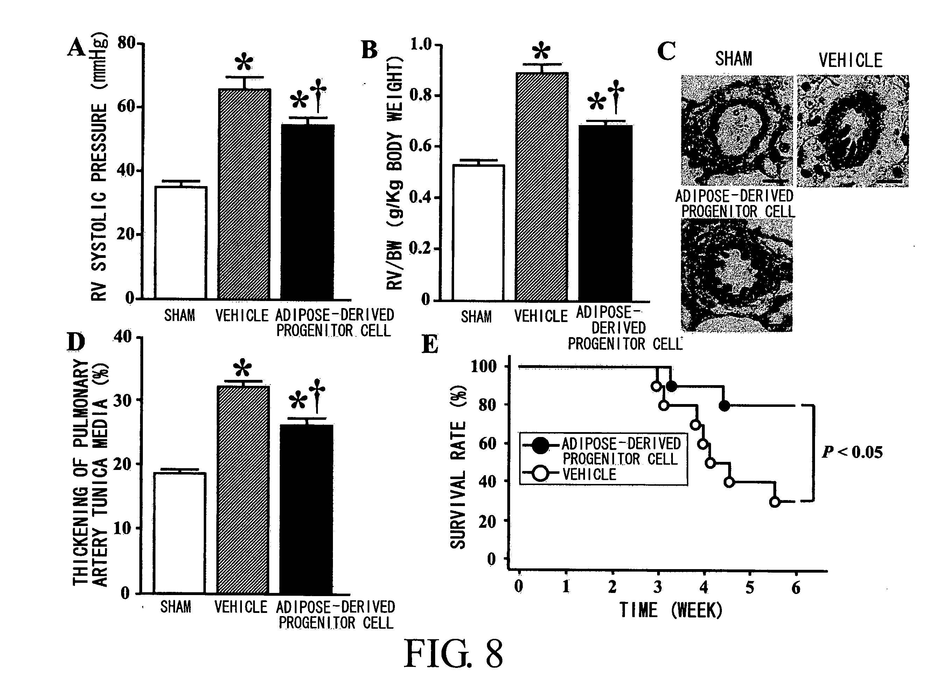

Fig. 8 shows the effect of the transplantation of adipose-derived progenitor cells on the right ventricle (RV) systolic pressure (A), and the ratio of RV weight to body weight (RV/BW) (B). Values are means ± SEM. * P<0.05, as compared to sham; † P<0.05, as compared to vehicle. C shows representative photographs of the peripheral pulmonary artery after three weeks from MCT injection. Scale bar: 10 µm. D is a graph showing the quantitative analysis on the rate of change of wall thickness of the peripheral pulmonary artery. Values are means ± SEM. * P<0.05, as compared to sham; † P<0.05, as compared to vehicle. E shows Kaplan-Meier survival curves, showing that the survival rate of the adipose-derived progenitor cell group (closed circle) is significantly higher than that of the vehicle group (open circle) (logrank test, P<0.05). Sham, sham-treated rats administered with medium; Vehicle, MCT rats administered with medium; Adipose-derived progenitor cell, MCT rats administered with adipose-derived progenitor cells; RV, right ventricle; RV/BW, ratio of RV weight to body weight; LV + S/BW, ratio of weight of left ventricle + septum to body weight. Values are means ± SEM. * P<0.05, as compared to sham; † P<0.05, as compared to vehicle.

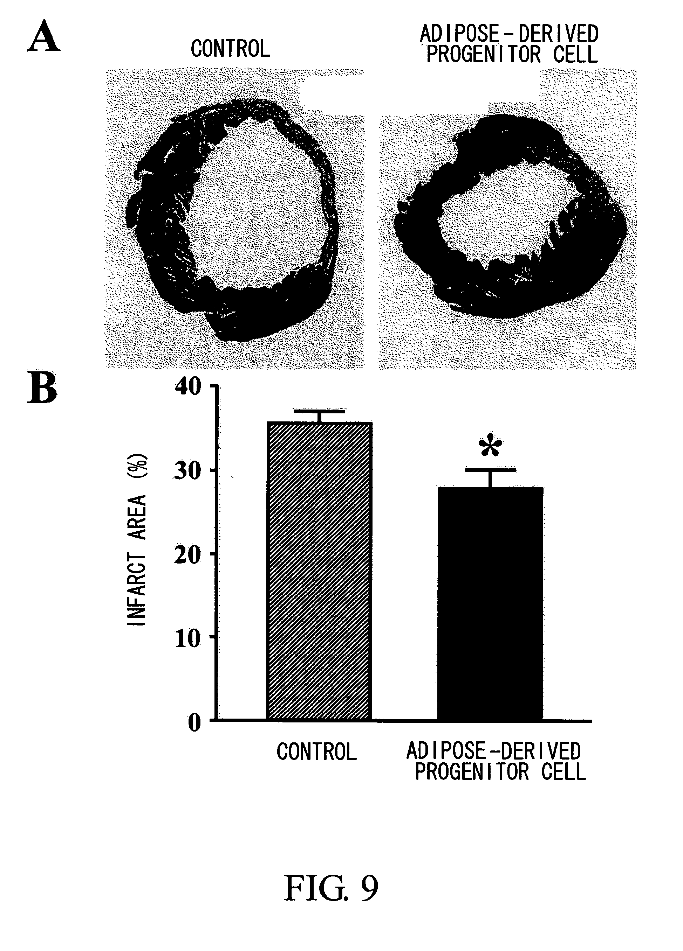

Fig. 9 shows the effect of the transplantation of adipose-derived progenitor cells four weeks after acute myocardial infarction. A shows representative photographs of infarct area of the control group and the adipose-derived progenitor cell transplanted group stained with Masson Trichrome. B is a graph showing the result of semiquantitative analysis which proves that the infarct size was remarkably decreased by transplantation of adipose-derived progenitor cells. Values are means ± SEM. * P<0.05, as compared to control.

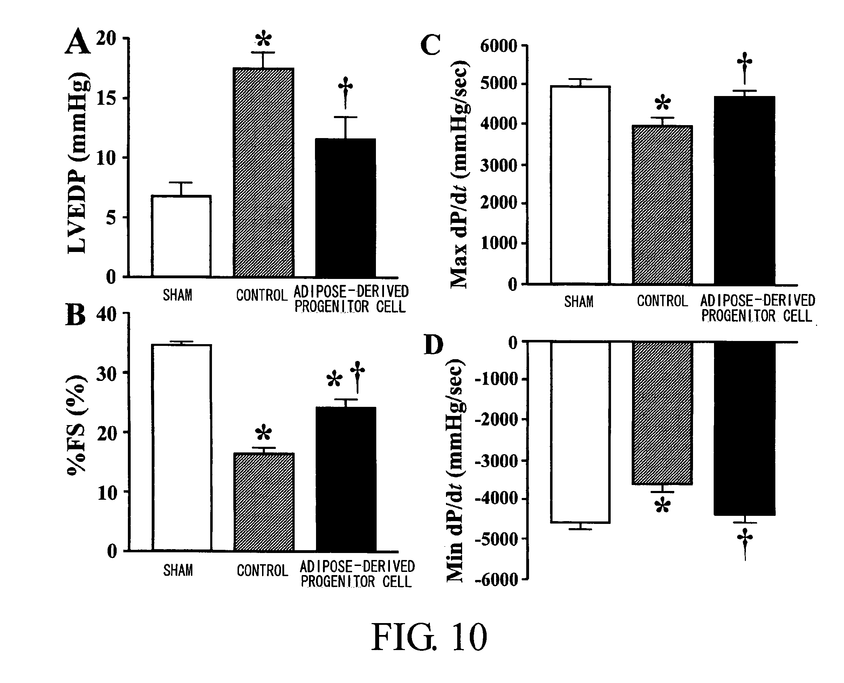

Fig. 10 shows the effect of transplantation of adipose-derived progenitor cells on hemodynamic parameters. A: LV end-diastolic pressure (LVEDP), B: LV fractional shortening (FS %), C: LV maximum dP/dt (Max dP/dt), and D: LV minimum dP/dt (Min dP/dt). Values are means ± SEM. * P<0.05, as compared to sham; † P<0.05, as compared to control.

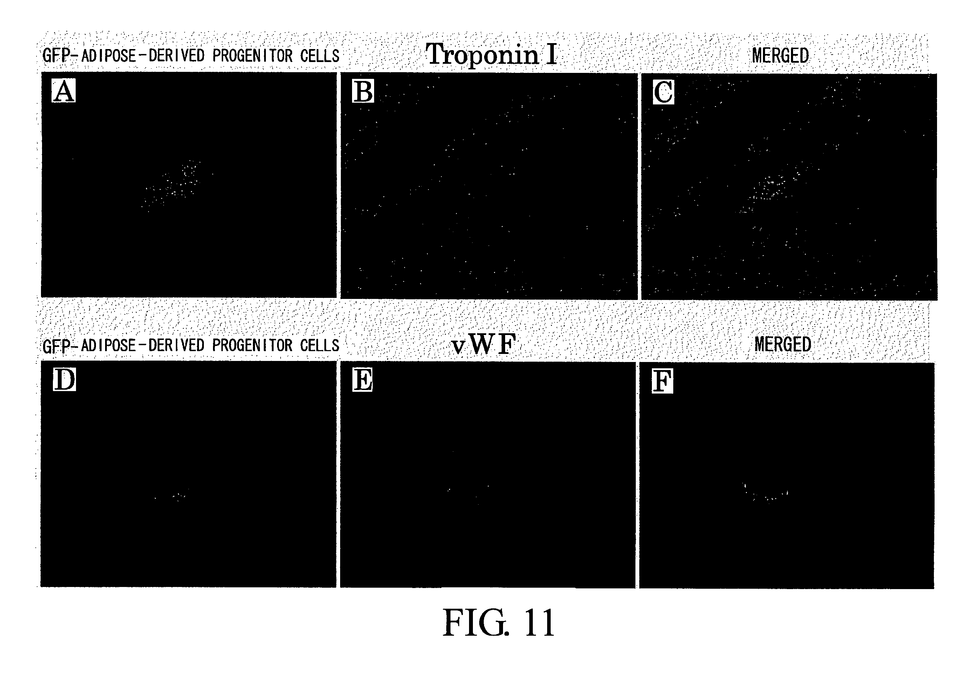

Fig. 11 shows the survival and differentiation of transplanted adipose-derived progenitor cells in the ischemic myocardium. A-C are photographs showing that GFP positive adipose-derived progenitor cells are cardiac troponin I positive. Magnification, x 400. D-F are photographs showing that GFP positive adipose-derived progenitor cells form vessel structure and vWF is stained. Magnification, x 630. The nuclei were stained with DAPI.

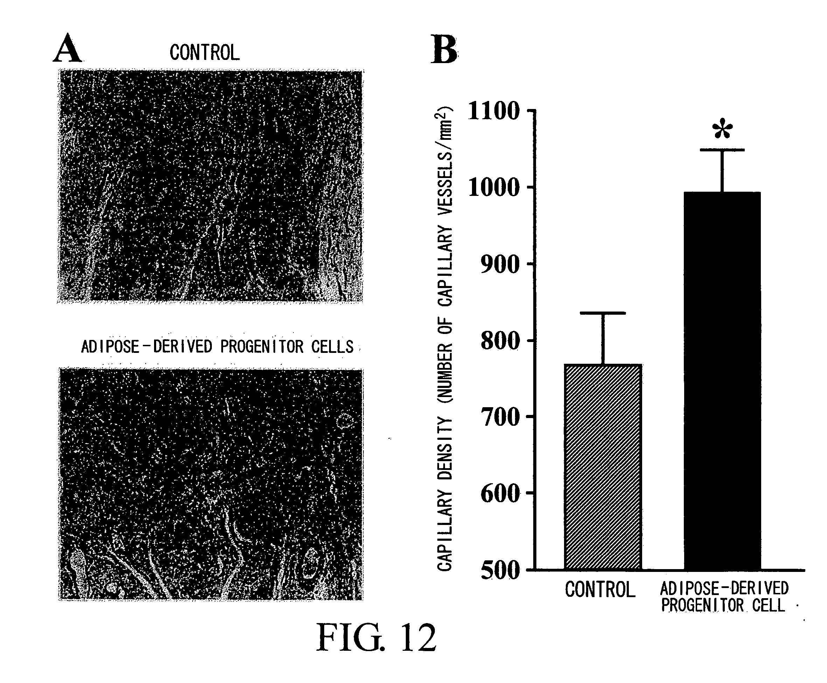

Fig. 12A shows photographs immunohistochemically showing vWF in the peri-infarct area. Magnification, x 200. B is a graph showing the quantitative analysis of the capillary density in the peri-infarct area. The capillary density in the adipose-derived progenitor cell group was remarkably higher compared with the control group. Values are means ± SEM. * P<0.05, as compared to sham; † P<0.05, as compared to control.

### Best Mode for Carrying Out the Invention

The present invention relates to methods for producing progenitor cells, comprising the step of culturing pluripotent cells in a medium containing a growth factor(s).

In the present invention, examples of "pluripotent cells" include adipose stromal cells, embryonic stem cells (ES cells), adult stem cells, mesenchymal stem cells, cord blood cells, and cells which have artificially acquired a pluripotency. However, any cell having an ability to differentiate into various types of cells can be included in the pluripotent cell of the present invention. A preferable example of the pluripotent cell of the present invention includes adipose stromal cells contained in adipose tissues. Moreover, adipose stromal cells differentiated from mature adipocytes can also be used as adipose stromal cells for the present invention. Preferably, the pluripotent cell of the present invention is derived from mammals; however, the origin is not specifically limited. The term "cells which have artificially acquired a pluripotency" includes for example somatic cells which have acquired a pluripotency by clone technologies including gene manipulations.

In the present invention, the "growth factor" includes adrenomedullin (AM) and vascular endothelial growth factor (VEGF). Each of these growth factors may be added to a medium either alone or as a mixture at the time of differentiation induction. For making the mixture, the concentrations of the respective growth factors are not specifically limited, and may be either the same concentration or different concentrations. Moreover, the respective growth factors may be added to a medium stepwise during culture.

In the present invention, the term "adrenomedullin" refers to a bioactive peptide with a powerful antihypertensive effect discovered in a human pheochromocytoma tissue extract using the increase of cAMP activity in thrombocytes as an indicator. The nucleotide sequence of an "adrenomedullin" precursor cDNA is shown in SEQ ID NO: 1, and the amino acid sequence of a protein encoded by the cDNA is shown in SEQ ID NO: 2.

"Adrenomedullin" precursors in the present invention can include naturally-occurring proteins which comprise an amino acid sequence with one or more amino acid substitutions, deletions, insertions, and/or additions in the amino acid sequence of SEQ ID NO: 2, and which are functionally equivalent to the protein comprising the amino acid sequence of SEQ ID NO: 2. Moreover, the "adrenomedullin" precursors can include proteins which are encoded by naturally-occurring DNAs that hybridize under stringent conditions with DNAs comprising the nucleotide sequence of SEQ ID NO: 1, and which are functionally equivalent to the protein comprising the amino acid sequence of SEQ ID NO: 2.

Active "adrenomedullin" is part of the "adrenomedullin" precursors, and comprises the amino acid sequence of SEQ ID NO: 3. Adrenomedullin used in the present invention preferably comprises the amino acid sequence of SEQ ID NO: 3, but is not limited thereto. It can include naturally-occurring proteins which comprise an amino acid sequence with one or more amino acid substitutions deletions, insertions, and/or additions in the amino acid sequence of SEQ ID NO: 3, and which are functionally equivalent to the protein comprising the amino acid sequence of SEQ ID NO: 3. The active "adrenomedullin" can also include proteins which are encoded by naturally-occurring DNAs that hybridize under stringent conditions with the DNA corresponding to positions 447 to 602 in the nucleotide sequence of SEQ ID NO: 1, and which are functionally equivalent to the protein comprising the amino acid sequence of SEQ ID NO: 3.

In the present invention, the number of mutated amino acids is not particularly limited, but is usually 30 amino acids or less, preferably 15 amino acids or less, and more preferably 5 amino acids or less (for example, 3 amino acids or less). Amino acid residues are preferably mutated to amino acids that conserve amino acid side chain properties. For example, examples of amino acids with amino acid side chain properties include: hydrophobic amino acids (A, I, L, M, F, P, W, Y, and V), hydrophilic amino acids (R, D, N, C, E, Q, G, H, K, S, and T), amino acids with aliphatic side chains (G A, V, L, I, and P), amino acids with hydroxyl-containing side chains (S, T, and Y), amino acids with sulfur-containing side chains (C and M), amino acids with carboxylic acid- and amide-containing side chains (D, N, E, and Q), amino acids with base-containing side chains (R, K, and H), and amino acids with aromatic-containing side chains (H, F, Y, and W) (amino acids are represented by one-letter codes in the parentheses). It is known to those skilled in the art that a polypeptide that comprises an amino acid sequence modified by a deletion, addition, and/or substitution with another amino acid of one or more amino acid residues maintains its biological activity.

In the present invention, the term "functionally equivalent" means that the protein has biological and biochemical functions equivalent to those of a protein of interest. In the present invention, biological and biochemical functions of a protein of interest can include the function to regenerate or repair a damaged tissue in a subject; the function to generate or regenerate peripheral vessels of the damaged tissue; and the function to generate or regenerate cells of the damaged tissue. Methods for measuring such functions can be, for example, methods described in the Examples, but are not limited thereto. Biological properties comprise expression site specificity and expression level.

Methods well known to those skilled in the art for preparing DNAs that encode "proteins which are functionally equivalent" to target proteins include methods that utilize hybridization or polymerase chain reaction (PCR) techniques. Namely, those skilled in the art can routinely isolate DNAs that are highly homologous to "adrenomedullin" by using the nucleotide sequence of "adrenomedullin" (SEQ ID NO: 1) or a part thereof as a probe, or by using oligonucleotides that specifically hybridize with the nucleotide sequence of "adrenomedullin" (SEQ ID NO: 1) as primers. DNAs encoding proteins that are functionally equivalent to "adrenomedullin" and which can be isolated by hybridization or PCR techniques are also comprised in the DNAs of the present invention.

In order to isolate such DNAs, the hybridization reaction is preferably performed under stringent conditions. In the present invention, "stringent hybridization conditions" refers to conditions of 6M urea, 0.4% SDS, and 0.5x SSC, or hybridization conditions with a stringency equivalent to these conditions. DNAs with higher homology may be isolated using conditions with higher stringency, for example, conditions of 6M urea, 0.4% SDS, and 0.1x SSC. The DNAs thus isolated are considered to have high homology with the amino acid sequence of the target protein at the amino acid level. High homology refers to a sequence identity of at least 50% or more, more preferably 70% or more, still more preferably 90% or more (for example, 95%, 96%, 97%, 98%, 99% or more) across the whole amino acid sequence. The identity of amino acid sequences and nucleotide sequences can be determined using the BLAST algorithm by Karlin and Altschul. Programs called BLASTN and BLASTX have been developed based on the BLAST algorithm. When analyzing nucleotide sequences using BLASTN, parameters are set, for example, to score = 100 and wordlength = 12. When analyzing amino acid sequences using BLASTX, parameters are set to, for example, score = 50 and wordlength = 3. When using BLAST and Gapped BLAST programs, the default parameters of each program are used. The specific procedures of these analyzing methods are known.

In the present invention, the term "vascular endothelial growth factor (VEGF)" refers to growth factors specific to vascular endothelial cells. VEGF binds to receptors of endothelial cells inside vessels to promote proliferation. VEGF is known to have five types of isoforms (VEGF121, 145, 165, 189, and 206) having different molecular weights, and the type of the VEGF to be added to a medium in the present invention is not specifically limited.

The origins of the above adrenomedullin and vascular endothelial growth factor are not specifically limited; however, they are preferably derived from mammals, more preferably a human.

In the culture method of the present invention, when adipose stromal cells are used, preculture may be performed prior to differentiation induction as described in the Examples; however this step may be omitted. In this preculture step, it is possible to proliferate undifferentiated adipose stromal cells while maintaining the pluripotency.

The preculture conditions are not specifically limited, and media and additives (preferably heparin or antibiotics) known to those skilled in the art can be used. The preculture period is also not specifically limited, a preferable example being 7 days to 28 days.

The cell culture method in the present invention may include: two-dimensional culture methods using culture dishes having different matrix coatings, or culture dishes where the presence/absence of matrix coatings are different; three-dimensional culture methods using a soft gel such as matrigel, a collagen sponge, and the like; or combinations thereof. However, culture dishes having different matrix coatings are preferred. In the preculture step, it is not necessary to use the culture dishes mentioned above.

Moreover, in methods of the present invention, when human-derived cells having a pluripotency other than adipose stromal cells are used, the culture dishes may be appropriately selected with reference to exemplified adipose stromal cells in Examples.

Furthermore, Examples of the present invention disclose detailed culture conditions for the preculture step and the culture step after differentiation induction. However, the culture conditions in the methods of the present invention are not limited to these specific conditions, and may be generally acceptable conditions.

The culture period in the methods of the present invention is not specifically limited. However, when adipose stromal cells are used in methods of the present invention, the period is preferably 7 to 28 days.

In the present invention, progenitor cells can be produced according to the above methods of differentiation induction. In the present invention, the progenitor cells are not specifically limited; however, vascular endothelial progenitor cells and myocardial progenitor cells can be preferably included.

Whether the differentiated cells produced in the present invention are progenitor cells or not, can be confirmed using cell markers for respective tissues or respective cellular functions as an index.

As cell markers for respective tissues, vascular endothelial cell markers and myocardial cell markers may be preferably used. Examples of vascular endothelial cell markers can include monoclonal antibodies: CD31 (clone TLD-3A12, Becton Dickinson), vascular endothelial (VE)-cadherin (clone F-8, Santa Cruz), CD45 (clone OX-1, Becton Dickinson), CD34 (clone ICO115, Santa Cruz), and rabbit polyclonal antibodies (Dako) to von Willebrand factor (vWF). Examples of myocardial cell markers can include troponin I, ANP, and connexin43.

The function of vascular endothelial progenitor cells can be confirmed by *in vitro* or *in vivo* confirmation of whether neovascularization is induced. For example, as described in Examples, vessel formation assay may be performed in a matrigel, or MCT rats serving as pulmonary hypertension model animals may be administered with cells of the present invention to confirm whether pulmonary hypertension is improved.

The function of myocardial progenitor cells can be confirmed by verifying *in vitro* or *in vivo* whether the neogenesis of myocardial cells is induced. For example, as described in Examples, it may be confirmed whether self-beating of cultured cells occurs when cocultured with myocardial cells from a neonatal rat, or whether myocardial cells are newly generated by administering cells of the present invention to myocardial infarction model rats.

In methods of the present invention, even when human-derived pluripotent cells other than adipose stromal cells are used, produced progenitor cells can be confirmed by detection methods known to those skilled in the art (detection methods using respective cell markers and assay methods specific to respective cells).

The present invention provides progenitor cells produced by methods of the present invention. The progenitor cells produced by methods of the present invention can be more readily produced *ex vivo* as compared to progenitor cells produced by conventional methods. Moreover, since the methods of the present invention can induce differentiation from adipose stromal cells and the like into progenitor cells, there is an advantage in that a progenitor cell amount necessary for therapy can be obtained using only a procedure that is low invasive to the patient. Accordingly, progenitor cells of the present invention are useful for example, in medical fields (such as the field of regenerative medicine).

The present invention relates to methods for regenerating or repairing damaged tissues in subjects, comprising the step of administering progenitor cells of the present invention to the subjects. In the present invention, the term "regenerating or repairing damaged tissues" more specifically includes newly generating or regenerating peripheral vessels of damaged tissues, and newly generating or regenerating cells of damaged tissues.

In the present invention, the term "subject" means an organism to be treated by the methods of the present invention, a body part of the organism (for example, organs such as brain, lung, heart, liver, and kidney, and blood vessels or tissues of an organ), or a part excised or excreted from the organism. The organism can be any animal (for example, humans, domestic animals, and wild animals) without particular limitation, but preferably comprises mammals, and more preferably humans. In the present invention, the term "subject" can also refer to a tissue that has received damage itself (a damaged tissue).

In the present invention, the term "administration" includes parenteral administration.

Parenteral administration includes administration in the form of an injectable agent. The injectable agent includes agents for subcutaneous injection, agents for intramuscular injection, agents for intraperitoneal injection, and the like. Local administration may be performed as a method for administering such injectable agents, targeting a part (a tissue such as an organ) inside the organism of interest, or vascular administration may be performed to circulate cells of the present invention throughout the whole organism. Simultaneous administration may be also performed targeting a plurality of sites. Furthermore, cells of the present invention may be locally administered to sites to be treated. For example, administrations can also be performed by means of intraoperative local infusions or the use of a catheter.

The dose varies depending on the age, gender, weight, and symptoms of patients, therapeutic effects, administration methods, treatment time, types of active ingredients contained in the cells and the like, and is not specifically limited.

Moreover, when administration is performed to a part of a organism extracted or excreted from the organism, cells of the present invention may be brought into "contact" with the part of the organism.

Furthermore, in the present invention, the "contact" is performed according to the condition of the organism. The "contact" can include for example disseminating cells of the present invention over the part of the organism, or adding cells of the present invention to a homogenate of the part of the organism, but is not limited thereto.

When methods of the present invention are practiced, cells of the present invention may be administered as a part of a pharmaceutical composition together with at least one well-known chemotherapeutic agent. In one embodiment, cells of the present invention and a well-known chemotherapeutic agent may be substantially simultaneously administered.

Moreover, cells of the present invention may be simultaneously administered with at least one already-known growth factor. The growth factor to be simultaneously administered is not specifically limited, but preferably includes adrenomedullin and vascular endothelial growth factor. The adrenomedullin and vascular endothelial growth factor may each be administered alone, or simultaneously.

Furthermore, it is also possible to administer cells of the present invention into a part of an organism extracted or excreted from the organism, and then place the part back into the organism from which the extraction or excretion was performed, or into another organism.

The term "damaged tissue" in the present invention can refer to a tissue that has received damage due to a certain reason. When an external or internal stimulus is applied to a normal cell, usually this does not immediately lead to cell damage and an adaptation phenomenon occurs, leading to atrophy, hypertrophy, and hyperplasia of cells/tissues. However, if the external or internal stimulus exceeds the adaptable range, morphological changes accompanying the stimulus, such as degeneration and necrosis, may take place; and such phenomena are defined as "damages" in the present invention.

In the present invention, the term "degeneration" in a damaged tissue refers to a change in a cell or tissue that is universally recognized as reversible cell damage. Some of the substances accumulated at the time of degeneration are normally present in a living body, and some are pathological substances not normally present. Degeneration is referred to as protein denaturation or fatty degeneration depending on the accumulated substance. Degenerate states include mucous degeneration, amyloid degeneration, degeneration as a result of α-1 antitrypsin deficiency, degeneration in uric acid metabolism disorders, hyaline droplet degeneration, dropsy-like degeneration, vacuolar degeneration, fibrinoid degeneration, and cornification.

In the present invention, the term "necrosis" refers to an irreversible change in cells or tissues. Necrotic states include coagulation necrosis (circulatory failure), colliquative necrosis, gangrene (a state in which bacterial infection is combined with necrosis), and apoptosis (physiological death occurred in tissues, not pathological death from cell damage).

In the present invention, the "damaged tissue in a subject" includes damaged tissues in diseases of the cranial-nerve system, endocrine system, circulatory organs, respiratory organs, digestive organs, urinary organs, skin and such.

The above-mentioned diseases include myocardial infarction, arrhythmia, arteriosclerosis, vasospasm, ischemic recirculation failure, pneumonia, infectious diseases, fibroid lung (adverse reaction of anticancer agents), ARDS, paraquat intoxication, smoking disorder, pulmonary emphysema, hyperoxia therapy, influenza, cerebral edema, cerebral infarction, cerebral hemorrhage, epilepsy, cerebrovascular spasm, Parkinson's disease, dysautonomia (Reilly phenomenon), late-onset neuropathy, spinal cord injury, neurogenic pulmonary edema, acute gastric mucosal disorder, gastric ulcer, ulcerative colitis, Crohn's disease, Behcet's disease, pneumonia, liver cirrhosis, drug-induced hepatopathy, pathology of liver transplantation, various types of clinical conditions of jaundice, pancreatitis, glomerulonephritis, hemolytic renal disorder, drug-induced renal disorder, burn, solar dermatitis, atopic dermatitis, skin ulcer, retinal degeneration, cataract, and corneal tumor. Preferably, the diseases include intractable cardiopulmonary diseases, peripheral vascular diseases, renal diseases, liver diseases, and cerebrovascular diseases, and more preferably, pulmonary hypertension, or ischemic heart disease (myocardial infarction or angina pectoris).

More specifically, the "damaged tissue in a subject" of the present invention includes damaged tissues in alveoli and pulmonary blood vessels, or myocardial cells.

The term "regenerate or repair" in the present invention refers to returning the above-mentioned damaged tissue to the state before degeneration, or newly generating a damaged tissue that has necrotised. For example, generating or regenerating peripheral blood vessels of damaged tissues in a subject or generating or regenerating cell tissues of damaged tissues is also encompassed in the "regeneration or repair" of the present invention.

The period of regeneration or repair of a damaged tissue is not particularly limited, and may be temporary or a fixed period of time. Moreover, although tissue regeneration or repair may be partial, it is preferable that regeneration or repair progresses fully, that is, until the tissues are recovered completely to their original condition.

More specific examples of "regeneration or repair" include repair of degenerated peripheral vessels or regeneration of new blood vessels that substitute for necrotised peripheral vessels, in pulmonary alveoli or pulmonary vessels in a state of emphysema. Cases where pulmonary hypertension is improved as a result can also be interpreted as actual repair/regeneration of pulmonary tissue. Moreover, examples thereof also include repair of degenerated myocardial cells or peripheral vessels, in myocardial infarction or angina pectoris. Cases where symptoms of myocardial infarction or angina pectoris are improved as a result can also be interpreted as actualrepair/regeneration of the cardiac tissue.

The present invention relates to pharmaceutical agents which regenerate or repair damaged tissues, comprising progenitor cells of the present invention as an active ingredient. Moreover, the present invention relates to pharmaceutical agents which newly generate or regenerate peripheral vessels in damaged tissues, or pharmaceutical agents which newly generate or regenerate cells in damaged tissues, comprising progenitor cells of the present invention as an active ingredient.

Pharmaceutically acceptable carriers such as preservatives and stabilizers may be added to the pharmaceutical agents of the present invention. The term "pharmaceutically acceptable" material refers to materials which do not have the abovementioned activities by themselves, and those that can be administered together with the pharmaceutical agent mentioned above.

When an injectable agent is prepared, pH-regulators, buffers, stabilizers, preservatives, and the like are added as required, and formed into a formulation for subcutaneous, intramuscular, or intravenous injection, by ordinary methods. Moreover, a single dose may be stored in a container, or a plurality of doses may be stored in the same container.

All prior art documents cited in the present specification are incorporated herein by reference.

### [EXAMPLES]

Herein below, the present invention will be further specifically described with reference to Examples, but it is not to be construed as being limited to these Examples. In the present Examples, data are expressed as means ± SEM. Parameter comparisons among groups were performed by one-way ANOVA test, followed by Newman-Keuls test. Moreover, comparisons of sequential changes of parameters among groups were performed by two-way ANOVA test with respect to replicate measurements, followed by Newman-Keuls test. The survival curve was formed by Kaplan-Meier method, and comparison was made by logrank test. Values of P<0.05 were considered as statistically significant.

### [Example 1] Isolation and culture of adipose stromal cells from adipose tissue

Male Wistar rats weighing 90 to 110 g were used for the present Example. The rats were operated to extract subcutaneous adipose tissues from tiny incisions in both sides of the abdomen. The adipose tissues were homogenated and put in a phosphate-buffered saline containing 0.1 mg/ml type I collagenase (Worthington Biochemical). The mixture was gently stirred to effect a digestion treatment at 37°C for one hour. Undigested fragments were removed by filtration using a 25 µm filter. Then, centrifugation (at 2000 rpm for 10 minutes) was performed to isolate mature adipocytes from the pellet of adipose stromal cells. As previously reported (Bjorntorp P, et al. J Lipid Res. 1978; 19:316-324), the supernatant was removed, and the cell pellet was resuspended, seeded in a normal culture dish, and cultured in an α medium containing 10% fetal bovine serum (FCS), heparin, and antibiotics.

On day 7 of the culture, adhesive adipose stromal cells were subcultured, and divided into four groups as follows: adipose stromal cells cultured in an α medium containing 10% FCS and antibiotics (control medium) (control group); adipose stromal cells cultured in a control medium containing 10⁻⁷ mol/l AM (AM group); adipose stromal cells cultured in a control medium containing 20 ng/ml VEGF (VEGF group); and adipose stromal cells cultured in a control medium containing both of 10⁻⁷ mol/l AM and 20 ng/ml VEGF (AM + VEGF group).

The adipose stromal cells on day 7 of culture exhibited a spindle-shape, while the shape of cells in the control medium remained unchanged up to day 28 (Fig. 1). On the other hand, adipose stromal cells cultured together with AM and VEGF exhibited a cobblestone appearance on day 28 (Fig. 1).

### [Example 2] Analysis by fluorescence-activated cell sorter

The endothelial phenotype of the cultured cells were confirmed by proving the existence of endothelial cell-specific markers such as VE-cadherin, CD31, and vWF. Specifically, at the time points of day 7, 14, 21, and 28 of culture, adipose stromal cells of each group were analyzed by fluorescence-activated cell sorting method (FACS; FACS SCAN flow cytometer, Becton Dickinson). These adipose stromal cells were incubated at 4°C for 30 minutes with mouse monoclonal antibodies against CD31 (clone TLD-3A12, Becton Dickinson), vascular endothelial (VE)-cadherin (clone F-8, Santa Cruz), CD45 (clone OX-1, Becton Dickinson), and CD34 (clone ICO115, Santa Cruz), respectively, and with rabbit polyclonal antibodies (Dako) against von Willebrand factor (vWF). A permeabilization buffer (Santa Cruz) was used for intracellular staining of VE-cadherin. Antibodies having the same isotype were used as a control.

These results showed that the majority of adipose stromal cells were negative for VE-cadherin (6.4±1.8% on day 7 and 7.2±2.4% on day 28) (Fig. 2-1, top graph). However, it was found that adipose stromal cells expressing VE-cadherin were gradually increased by culturing with either AM or VEGF (respectively 28±7% and 22±3% on day 28). Moreover, importantly, it was found that adipose stromal cells expressing VE-cadherin were further increased by culturing with both of AM and VEGF (49±7% on day 28). It was found that the number of adipose stromal cells expressing CD31 was gradually decreased in the control medium (from 6.4±1.2% on day 7 to 2.6±0.7% on day 28), but was significantly increased when cultured with either AM or VEGF (respectively 16±11% and 11±7% on day 28) (Fig. 2-1, bottom graph). It was found that the number of adipose stromal cells expressing CD31 was further increased by the combination of AM and VEGF (26±11% on day 28), and similarly, the number of adipose stromal cells expressing vWF was also increased from 12±3% on day 7 to 70±13% on day 28 by culturing with both of AM and VEGF (Fig. 2-2). On the other hand, the expression of CD34 and CD45 was not changed in all groups.

From the above results showing that the expression of VE-cadherin or CD31 was increased in culture, it was found that, similarly to VEGF, AM has an ability to enhance the differentiation of adipose stromal cells into an endothelial cell lineage.

Furthermore, the involvement of AM in the differentiation signaling was examined as previously reported (Kim W, et al. FASEB J. 2003;17:1937-1939) by culturing adipose stromal cells in the presence or absence of 30 nmol/l wortmannin (Sigma) serving as a PI3K inhibitor from day 21 to day 28 while replacing the medium every 24 hours. The expression of VE-cadherin was analyzed by FACS on day 28.

As a result, it was found that the enhancement of the VE-cadherin expression by means of AM, VEGF, or AM + VEGF was attenuated by administering the PI3K inhibitor (Fig. 3). It was revealed that the number of adipose stromal cells expressing VE-cadherin was not significantly different among the three groups: 22±3% with 20 ng/ml VEGF, 25±6% with 50 ng/ml VEGF, and 20±4% with 100 ng/ml VEGF.

Prior investigations show that downstream factors in PI3K signaling play an important role in the VEGF-induced endothelial differentiation in embryonic stem cells (Miyashita H, et al. Blood. 2002;99:3241-3249, Yamazaki T, et al. Blood. 2004;104:2345-2352). In the present invention, culture with a PI3K inhibitor inhibited the increase in the number of adipose stromal cells expressing VE-cadherin in the presence of AM or VEGF. These results suggest that PI3K signaling plays an important role in the AM- or VEGF-induced differentiation of adipose stromal cells into endothelial cells.

Moreover, even a relatively high concentration of VEGF (more than 20 ng/ml) did not increase the number of adipose stromal cells expressing VE-cadherin, whereas the combination of AM and VEGF induced the differentiation of adipose stromal cells into endothelial cell lineages more potently as compared to the single use of either AM or VEGF. These results suggest that AM plays an important role in the enhancement of VEGF-induced endothelial differentiation of adipose stromal cells.

### [Example 3] Measurement of neovascularization in matrigel

The formation of vessel-like structure by adipose stromal cells, that is, whether or not adipose stromal cells induce neovascularization, was evaluated in a basement membrane matrix preparation (matrigel). The cultured adipose stromal cells of each group were seeded in a 12-well plate coated with a matrigel (Becton Dickinson) at 2.0 x 10⁵ cells/well, and incubated at 37°C for 6 hours. Vessel formation was observed using an optical microscope, and the images were imported into a computer system. The pixel analysis of the vessel-formed area was performed as previously reported (Miura S, et al. Arterioscler Thromb Vasc Biol. 2003;23:802-808). Briefly, an image of this area was converted into a black-scale and image processing was performed to calculate total pixels. The number of pixels was measured in five different areas of each sample, and the mean value thereof was obtained.

As a result, in the AM group and the VEGF group, 1.9 fold and 1.6 fold increases in vessel formation were detected respectively, as compared to the control group (Figs. 4A and B). More importantly, further increase in vessel formation was detected in the AM + VEGF group. The quantification showed that the vessel formation of the AM + VEGF group was 2.3 times greater than that of the control group, and was significantly greater than that of either the AM group or the VEGF group.

From the above results, it was revealed that AM and VEGF both singly enhanced the neovascularization-promoting ability of adipose stromal cells, and the combination of AM and VEGF further enhanced the neovascularization-promoting ability as compared with the single use of either AM or VEGF.

### [Example 4] Detection of inhibitory effect of AM against adipocyte formation

The inhibitory effect of AM against adipocyte formation was examined.

Adipose stromal cells of each group on day 7and day 28 were resuspended in a Dulbecco's Modified Eagle Medium (DMEM: standard medium) containing 10% FCS and antibiotics, and were seeded in wells having a diameter of 22 mm at 1.2 x 10⁵ cells per well. The adipose stromal cells were differentiated into adipocytes by culturing in a standard medium containing 0.5 mmol/l 3-isobutyl-1-methylxanthine and 1 µmol/l dexamethasone for 48 hours, followed by culturing in a standard medium containing 10 µg/ml insulin for another 48 hours. The cells were kept in a standard medium containing no additives for 48 hours, and then stained with an oil red O solution. In order to measure the lipid accumulation by the quantification with oil red O staining, as previously reported (Landsberg RL, et al. Proc Natl Acad Sci USA. 2003;100:2456-2461., Selvarajan S, et al. Nat Cell Biol. 2001;3:267-275), isopropyl alcohol was added to the stained culture dish, then the extracted dye was immediately collected by gentle pipetting, and the absorbency at 490 nm was monitored with a spectrophotometer.

The results showed that the oil red O staining of adipose stromal cells on day 7 which have differentiated into adipocytes under adipocyte formation conditions, was 2.2 times greater than that of adipose stromal cells on day 7 which did not form adipocytes (Figs. 5A and B). Adipose stromal cells on day 28 in the control medium were differentiated substantially at the same level as adipose stromal cells on day 7. However, interestingly, it was revealed that the adipose conversion of adipose stromal cells on day 28 was almost completely inhibited by AM, VEGF, and AM + VEGF, even under the adipocyte formation conditions.

The evaluation of adipocyte formation showed that the adipose stromal cells cultured together with AM and/or VEGF were not differentiated into adipocytes even though adipocyte formation was induced. However, the adipose conversion ability of adipose stromal cells was maintained throughout the long-term culture. The above results suggest that adipose-derived progenitor cells have the potential to induce neovascularization *in vivo,* without accompanying adipose conversion.

### [Example 5] Introduction of adipose-derived progenitor cells into monocrotaline (MCT) rats

Monocrotaline (MCT), serving as a pulmonary hypertension inducer, induces pulmonary endothelial injuries and decreases the number of pulmonary capillaries (Rosenberg H, et al. Am J Physiol. 1988;255:H1484-H1491., Schraufnagel DE, et al. Am Rev Respir Dis. 1989;140:1405-1409), which contributes to the occurrence of MCT-induced pulmonary hypertension. Therefore, the present inventors examined the effect of adipose-derived progenitor cells on rats in which pulmonary hypertension had been artificially developed by administering MCT.

Adipose stromal cells obtained from peripheral adipose tissue were cultured together with 10⁻⁷ mol/l AM and 20 ng/ml VEGF for three weeks. The present inventors defined the cells induced by this culture of adipose stromal cells as adipose-derived progenitor cells. On the day 18 of culture, rats were randomly injected subcutaneously with 60 mg/kg MCT (MCT rat group) or 0.9% saline (sham-treated rat group). After three days from the MCT injection (on the 21 st day of culture), 1.5 x 10⁶ autologous adipose-derived progenitor cells or a medium (each 500 µL) were intravenously administered to the left jugular vein. The sham-treated rats were also intravenously administered with 500 µL of medium in the same manner. In order to obtain the maximum effect of transplantation based on the dose-response experiment, 1.5 x 10⁶ cells were used per rat. This protocol resulted in the following three groups:

MCT rats administered with adipose-derived progenitor cells (adipose-derived progenitor cell group, n = 10), MCT rats administered with medium alone (vehicle group, n = 10), and sham-treated rats administered with medium alone (sham-treated group, n = 10).

### [Example 6] Immunofluorescence staining and immunohistochemical staining

After three days from the 60 mg/kg MCT injection, 1.5 x 10⁶ autologous adipose-derived progenitor cells labeled with red fluorescent dye (PKH26, Sigma) were intravenously administered. After three weeks from the MCT injection, the lung was extracted and frozen. The frozen sections were subjected to immunofluorescence staining using vWF monoclonal antibodies (Dako) and corresponding FITC-labeled secondary antibodies (Dako).

By 18 days from the intravenous administration of adipose-derived progenitor cells, the adipose-derived progenitor cells had been incorporated into theMCT rats' pulmonary arteriolar wall constituting pulmonary vessels (Fig. 6A). The transplanted adipose-derived progenitor cells were distributed in the pulmonary tissue (Fig. 6B). Furthermore, some transplanted adipose-derived progenitor cells were stained with vWF, showing participation in vasculogenesis of the pulmonary artery (Fig. 6C).

4 µm-thick paraffin sections were prepared from the right lung of individual rats from the respective groups. The sections were subjected to immunohistochemical staining using rabbit polyclonal antibodies (DAKO) produced against vWF. The numbers of pulmonary alveoli and vWF positive capillary vessels (diameter: less than 100 µm) were calculated. The capillary density was expressed as the number of capillary vessels per 100 pulmonary alveoli as previously reported (Itoh T, et al. Am J Respir Crit Care Med. 2004; 170:1204-1211).

The results revealed that the capillary density was significantly decreased after MCT injection, but was significantly increased after the transplantation of adipose-derived progenitor cells into MCT rats (Figs. 7A and B).

The above results suggested that some introduced adipose-derived progenitor cells were differentiated into mature endothelial cells to induce endothelial cells.

### [Example 7] Effect of adipose-derived progenitor cells on pulmonary hypertension

Three weeks after the MCT injection, a hemodynamic test was performed. To measure the heart rate and the mean arterial pressure, a polyethylene catheter (PE-50) was inserted into the right carotid artery. To measure the RV pressure, a polyethylene catheter (PE-50) was inserted through the right jugular vein into the right ventricle (RV). Then, 2 mmol of potassium chloride was injected through the catheter to thereby induce cardiac arrest. The cardiac ventricles and the lungs were extracted and isolated. As previously reported (Nagaya N, et al. Circulation. 2000;102:2005-2012), the ratio of RV weight to body weight (RV/BW) and the ratio of the weight of left ventricle + septum to the body weight (LV + S/BW) were calculated as an index of ventricular hypertrophy. All protocols were based on the guidelines of the Animal Care and Ethics Committee of the National Cardiovascular Center Research Institute (Osaka, Japan).

The above results revealed that RV systolic pressure was significantly increased after three weeks from the MCT injection (Fig. 8A). However, this increase was significantly attenuated in the adipose-derived progenitor cell group (-17%). Similarly, the increase in RV/BW in MCT rats was significantly attenuated in the adipose-derived progenitor cell group (-23%) (Fig. 8B). The heart rate and the mean arterial pressure were not significantly different among the three groups (Table 1).

**[Table 1]**

| | Sham | Vehicle | Adipose-derived progenitor cells |
|---|---|---|---|
| n | 10 | 10 | 10 |
| Body weight, g | 268 ± 2 | 221 ± 5* | 242 ± 6*† |
| Heart rate, bpm | 396 ± 9 | 406 ± 9 | 410 ± 6 |
| Mean arterial pressure, mmHg | 119 ± 3 | 101 ± 6 | 106 ± 7 |
| RV systolic pressure, mmHg | 35 ± 2 | 66 ± 4* | 55 ± 3*† |
| RVBW, g/kg body weight | 0.52 ± 0.01 | 0.88 ± 0.03* | 0.68 ± 0.02*† |
| LV+S/BW, g/kg body weight | 1.97 ± 0.02 | 2.51 ± 0.07* | 2.32 ± 0.06*† |

### [Example 8] Morphometric analysis of pulmonary artery

4 µm-thick paraffin sections were prepared from the center of the right lung, and stained with hematoxylin and eosin for optical microscopic examination. The outer diameter and the inner wall thickness of the pulmonary arteriola were measured in 20 muscular arteries (within a range of 25 to 100 µm in the outer diameter). The inner wall thickness was expressed as follows: wall thickness = ([inner wall thickness x 2]/outer diameter) x 100. As previously reported (Itoh T, et al. Am J Respir Crit Care Med. 2004;169:34-38), lung sections were obtained from individual rats of three groups (respectively, n = 5).

In these results, representative photographs showed that the hyperplasia of the pulmonary vascular wall caused by MCT injection was attenuated in the adipose-derived progenitor cell group as compared with the vehicle group (Fig. 8C). Quantitative analysis showed a significant increase in the wall thickness percentage following MCT injection; however it was revealed that this change was improved in the adipose-derived progenitor cell group (Fig. 8D).

### [Example 9] Analysis of rat survival time

After three days from MCT injection, twenty rats were intravenously injected with 1.5 x 10⁶ adipose-derived progenitor cells derived from the autologous adipose tissue (adipose-derived progenitor cell group, n = 10), or a medium (vehicle group, n = 10). The survival time was evaluated from the date of MCT injection to the date of death of rat or to the sixth week after MCT injection.

In these results, the Kaplan-Meier survival curve showed that the survival rate of MCT rats transplanted with adipose-derived progenitor cells was significantly higher than that of the group administered the medium (Fig. 8E).

From the above, it is thought that vascular endothelial cells induced by the introduction of adipose-derived progenitor cells play an important role in regulating pulmonary revascularization and improving pulmonary hypertension.

### [Example 10] Introduction of adipose-derived vascular progenitor cells into myocardial infarction model rats

Rats were subjected to thoracotomy, and then the anterior descending branch of the coronary artery was ligated to produce myocardial infarction. After several minutes, 5x10⁶ adipose-derived progenitor cells were infused into the myocardium of infarct border zone (adipose-derived progenitor cell group). Moreover, as a control group, similarly, after the myocardial infarct was produced, 100 µL of PBS was infused into the myocardium of infarct border zone (control group). The sham-treated group was subjected to thoracotomy, and then 100 µL of PBS was infused into the myocardium without producing a myocardial infarction therein (sham-treated group).

After four weeks from the myocardial infarct production, the therapeutic effect of the adipose-derived progenitor cells was evaluated.

The myocardial infarct sites (n = 5 in each group) of left cardiac ventricles from the rats of the control group and the adipose-derived progenitor cell group were fixed with 10% formalin, embedded in paraffin, and stained with Masson Trichrome. Moreover, the size of the myocardial infarct area was measured (Figs. 9A and B).

Next, in order to examine the hemodynamics after four weeks from the ligation of coronary arteries of rats from the control group and the adipose-derived progenitor cell group, LV end-diastolic pressure (LVEDP), LV fractional shortening (FS%), LV maximum dP/dt (Max dP/dt), and minimum dP/dt (Min dP/dt) serving as hemodynamic parameters were measured (Fig. 10A-D). A 1.5-Fr micronanometer-tipped catheter (Millar Instruments) was inserted into the right carotid artery to measure the mean arterial pressure. Next, the catheter was advanced into the left cardiac ventricle to measure the LV pressure. The measurement was recorded with a pressure transducer connected to a polygraph. The infarct size was measured as already reported (Chien YW. Et al., Am J Physiol Regul Integr Comp Physiol, 254:R185-91, 1988).

From the above results, reduction in the myocardial infarct area and hemodynamic improvement were recognized in the adipose-derived progenitor cell group, as compared with the control group. Moreover, improvement in cardiac function was recognized in the adipose-derived progenitor cell group by echocardiography.

### [Example 11] Differentiation of adipose-derived progenitor cells into myocardial cells on culture

Adipose stromal cells obtained from the peripheral adipose tissue of EGFP-transgenic rats were subjected to primary culture for one week, and then cultured in a medium added with 10⁻⁷ mol/l AM for another week, to produce adipose-derived progenitor cells. Moreover, myocardial cells were isolated from 1- or 2-day-old neonatal rats, and cultured. On the second day of culture, the adipose-derived progenitor cells were added thereto, to start a coculture. On the fifth day of coculture, the adipose-derived progenitor cells started self-beating. Since self-beating is a phenomenon specific to myocardial cells, it was confirmed that the adipose-derived progenitor cells have differentiated into myocardial cells by coculturing with myocardial cells from neonatal rats. The self-beating behavior of the adipose-derived progenitor cells was successfully captured into video images.

On the fifth day of coculture, cells in the culture dish were fixed with paraformaldehyde, and were subjected to fluorescent immunostaining with troponin I, ANP, and connexin 43 serving as myocardium-specific markers. By staining troponin I, ANP, and connexin 43 in red, double staining together with green adipose-derived progenitor cells were recognized. This suggested that the adipose-derived progenitor cells have differentiated into myocardial cells.

### [Example 12] Fluorescent immunostaining and immunohistochemical staining of myocardium sections after transplantation in myocardial infarction

Adipose stromal cells obtained from the peripheral adipose tissue of EGFP-transgenic rats were subjected to primary culture for one week, and then cultured in a medium added with 10⁻⁷ mol/l AM for another week, to produce adipose-derived progenitor cells. The adipose-derived progenitor cells were transplanted into the rats after myocardial infarction production. After two weeks, the hearts were extracted. The hearts were fixed with formalin, from which paraffin sections were prepared, and troponin I, connexin 43, desmin, and vWF were stained. The results of troponin I staining are shown in Figs. 11A-C, and the results of vWF staining are shown in Figs. 11D-F. The transplantation after the myocardial infarction suggested that the adipose-derived progenitor cells have differentiated into myocardial cells or vascular endothelial cells.

Moreover, paraffin sections were prepared from the myocardium of individual rats from the respective groups. The sections were subjected to immunohistochemical staining using antibodies (DAKO) against vWF (Fig. 12A). The number of vWF positive capillary vessels (diameter: less than 100 µm) was calculated (Fig. 12B). The results revealed that the capillary density in the infarct border zone was significantly lowered after myocardial infarct production, but was significantly increased after transplantation of adipose-derived progenitor cells. This suggested that transplantation of adipose-derived progenitor cells promotes vasculogenesis, bringing about a therapeutic effect following myocardial infarction.

### Industrial Applicability

Using the methods of the present invention, progenitor cell numbers required for therapy can be produced by culturing *ex vivo* a small amount of adipocytes obtained by a low invasive procedure. Accordingly, methods for producing cells of the present invention and the cell transplantation therapy using the produced cells are innovative therapeutic methods which have overcome the conventional problems in that it is not necessary to perform highly invasive procedures on patients.

Increase in vascular flow and neovascularization in ischemic lower limbs and ischemic myocardium, or regeneration of myocardium can be expected by transplanting or introducing progenitor cells of the present invention into damaged tissues and the like. Moreover, it is thought that, by using progenitor cells of the present invention for intractable diseases in the brain, lung, liver, kidney or the like, tissue repair is promoted through revascularization or cell regeneration, to thereby decrease the lesion and improve the function thereof. Furthermore, methods of the present invention become innovative therapeutic methods since autologous cells are used, causing less ethical problems and less concern of rejection reactions due to immune responses.

## Claims

1. A method for producing a progenitor cell, comprising the step of culturing a pluripotent cell in a medium containing a growth factor of any one of the following (i) to (iii):
(i) adrenomedullin (AM);
(ii) vascular endothelial growth factor (VEGF); and
(iii) adrenomedullin (AM) and vascular endothelial growth factor (VEGF).

2. The method of claim 1, wherein the pluripotent cell is an adipose stromal cell, an embryonic stem cell, an adult stem cell, a mesenchymal stem cell, a cord blood cell, or a cell which has artificially acquired a pluripotency.

3. The method of claim 1, wherein the pluripotent cell is an adipose stromal cell contained in an adipose tissue.

4. The method of any one of claims 1 to 3, wherein the pluripotent cell is derived from a mammal.

5. A progenitor cell produced by a method of any one of claims 1 to 4.

6. A method for regenerating or repairing a damaged tissue in a subject, comprising the step of administering the progenitor cell of claim 5 to the subject.

7. A method for newly generating or regenerating a peripheral vessel of a damaged tissue in a subject, comprising the step of administering the progenitor cell of claim 5 to the subject.

8. A method for newly generating or regenerating a cell of a damaged tissue in a subject, comprising the step of administering the progenitor cell of claim 5 to the subject.

9. The method of any one of claims 6 to 8, comprising the step of administering a growth factor simultaneously with a progenitor cell to the subject.

10. The method of claim 9, wherein the growth factor is any one of following (i) to (iii):
(i) adrenomedullin (AM);
(ii) vascular endothelial growth factor (VEGF); and
(iii) adrenomedullin (AM) and vascular endothelial growth factor (VEGF).

11. The method of any one of claims 6 to 10, wherein the damaged tissue is a tissue damaged by an ischemic heart disease or pulmonary hypertension.

12. A pharmaceutical agent which regenerates or repairs a damaged tissue, comprising the progenitor cell of claim 5 as an active ingredient.

13. A pharmaceutical agent which newly generates or regenerates a peripheral vessel of a damaged tissue, comprising the progenitor cell of claim 5 as an active ingredient.

14. A pharmaceutical agent which newly generates or regenerates a cell of a damaged tissue, comprising the progenitor cell of claim 5 as an active ingredient.

15. The pharmaceutical agent of any one of claim 12 to 14, wherein the damaged tissue is a tissue damaged by an ischemic heart disease, peripheral artery obstruction, or pulmonary hypertension.
